(19) Europäisches Patentamt
European Patent Office
Office européen des brevets

(11) Numéro de publication: **0 139 552 B1**

(12) **FASCICULE DE BREVET EUROPEEN**

(45) Date de publication du fascicule du brevet: **08.02.89**

(51) Int. Cl.⁴: **G 01 N 33/535**, G 01 N 33/68, G 01 N 33/88, G 01 N 33/78

(21) Numéro de dépôt: **84401664.2**

(22) Date de dépôt: **13.08.84**

(54) **Composé marqué par l'acétylcholinestérase d'electrophorus electricus, son procédé de préparation et son utilisation comme marqueur dans des dosages enzymo-immunologiques.**

(30) Priorité: **17.08.83 FR 8313389**

(43) Date de publication de la demande: **02.05.85 Bulletin 85/18**

(45) Mention de la délivrance du brevet: **08.02.89 Bulletin 89/6**

(84) Etats contractants désignés: **BE CH DE FR GB IT LI LU NL SE**

(56) Documents cités:
**EP-A- 0 037 110**
**DE-A- 3 227 474**
**GB-A- 2 090 598**
**US-A- 4 152 411**

**J.Clin.Chem.Clin.Biochem., vol. 18 (1980), 197-208**
**Clinica Chimica Acta, 81 (1977) 1-40**
**Z.Anol.Chem. 279 (2), 1976-146**
**Bioch.Biophys.Res.COMM 1987 (469-476)**
**Trends in cluster headache pp. 125-134**
**CHEMICAL ABSTRACTS, volume 97, no. 25, 20 décembre 1982, page 121, abrégé 208627v, (COLUMBUS, OHIO, US); Y. HAYASHI et al.: "Enzyme**

(73) Titulaire: **COMMISSARIAT A L'ENERGIE ATOMIQUE, 31/33, rue de la Fédération, F-75015 Paris (FR)**

(72) Inventeur: **Grassi, Jacques, 19, rue Arthur Croquette, F-94220 Charenton le Pont (FR)**
Inventeur: **Pradelles, Philippe, 17, rue Pierre et Marie Curie, F-91160 Longjumeau (FR)**

(74) Mandataire: **Mongrédien, André et al, c/o BREVATOME 25, rue de Ponthieu, F-75008 Paris (FR)**

(56) Documents cités: (suite)
**immunoassay of PGF2alpha", & Methods Enzymol. 1982, 86(Prostaglandins Arachidonate Metab.), 269-73**

**Le dossier contient des informations techniques présentées postérieurement au dépôt de la demande et ne figurant pas dans le présent fascicule.**

**EP 0 139 552 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet un composé marqué par une enzyme, son procédé de préparation et son utilisation comme marqueur dans des dosages enzymoimmunologiques, sous la forme par exemple d'antigène marqué, d'haptène marqué ou d'anticorps marqué.

On sait qu'un antigène peut induire chez un vertébré supérieur la production d'anticorps spécifiques capables de former avec l'antigène un complexe réversible à haute énergie de liaison. Les haptènes sont des substances de bas poids moléculaire qui ne provoquent pas la formation d'anticorps lorsqu'on les injecte à un animal, mais qui réagissent avec les anticorps. Ces anticorps dirigés contre les haptènes peuvent être produits lorsqu'on injecte à un animal ou à l'homme, un composé conjugué par exemple de l'haptène et d'une protéine.

Les réactions antigène-anticorps ou haptène-anticorps peuvent être utilisées pour mesurer les concentrations en haptènes, en antigènes ou en anticorps d'un milieu donné, en utilisant des techniques immunoanalytiques telles que les techniques enzymoimmunologiques. Dans ce dernier cas, la réaction met en jeu un anticorps, un antigène ou un haptène et une molécule marquée par une enzyme qui peut être soit l'antigène, soit l'anticorps, soit l'haptène.

Selon la nature de la molécule qui est marquée par l'enzyme, on distingue deux types de dosage immunologique. Lorsque la molécule marquée est l'antigène ou l'haptène, on parlera de «dosage par compétition» ou de «dosage en quantité limitée de réactif». Dans ce cas, le principe du dosage repose sur la compétition entre l'antigène ou l'haptène marqué, d'une part, et l'antigène ou l'haptène, d'autre part, vis-à-vis d'un nombre limité de sites anticorps. De ce fait, pour le dosage immunologique, il faut établir une courbe d'étalonnage en faisant réagir des quantités constantes d'anticorps et de molécules marquées d'antigène ou d'haptène en quantités limitantes, avec des quantités variables d'antigène ou d'haptène. Après établissement de l'équilibre antigène-anticorps, on applique au mélange, une méthode de fractionnement permettant de séparer le complexe marqué de l'antigène ou de l'haptène qui reste libre en solution et par mesure de l'activité enzymatique de la fraction liée (complexe) ou de la fraction libre (antigène ou haptène marqué), on peut déterminer l'activité qui correspond aux différentes concentrations en antigène ou en haptène et établir la courbe d'étalonnage. Une fois cette courbe établie, on peut déterminer la concentration en antigène ou en haptène d'un échantillon en appliquant le même processus et en se reportant à la courbe d'étalonnage pour obtenir la concentration en haptène ou en antigène qui correspond à l'activité enzymatique trouvée lors du dosage.

Lorsque la molécule marquée est l'anticorps, on parlera de méthodes «Immunométriques» ou de dosage «en excès de réactif». Parmi ces méthodes on peut distinguer plusieurs variantes, les trois principales sont présentées ci-dessous.

I. Dosage immunométrique «classique»

Ce type de dosage est connu sous le nom de dosage Immunoradiométrique (IRMA) quand il met en jeu un anticorps marqué radioactivement.

Première étape:

Réaction de l'antigène avec un excès d'anticorps iodé

$$Ag + Ac^* \rightleftharpoons AgAc^* + Ac^*$$

Deuxième étape:

Adsorption de l'excès d'anticorps par un antigène immobilisé sur phase solide (excès d'antigène)

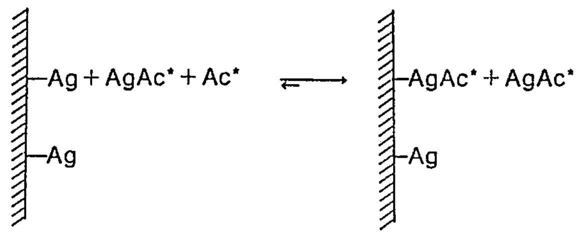


Troisième étape:

Séparation des deux phases et comptage du surnageant.

II. Dosage immunométrique à deux sites (méthode sandwich)

Première étape:

Réaction de l'antigène avec un premier anticorps adsorbé sur phase solide (excès d'anticorps)

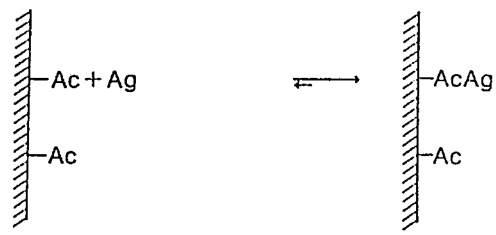


Deuxième étape:

Ajout d'un anticorps marqué reconnaissant un autre site de l'antigène (anticorps en excès)

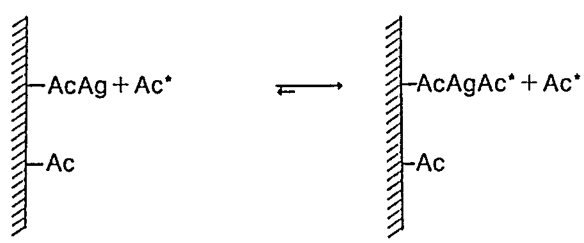


Troisième étape:

Elimination de l'excès d'anticorps marqué par lavage et comptage de la phase solide.

III. Dosage d'anticorps spécifique faisant intervenir un antigène immobilisé

Ce type de dosage ainsi que le dosage immunométrique à deux sites sont connus sous le nom

de méthode ELISA (Enzymo-Linked-Immumo-Sorbent-Assay) quand ils mettent en jeu un traceur enzymatique:

Première étape:

Réaction de l'anticorps spécifique avec l'antigène immobilisé (excès d'antigène).

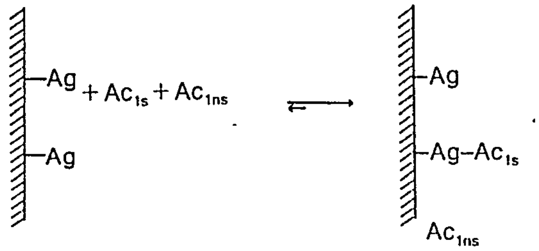


Deuxième étape:

Réaction du double anticorps marqué (ou de la protéine A marquée) avec l'anticorps fixé sur la phase solide.

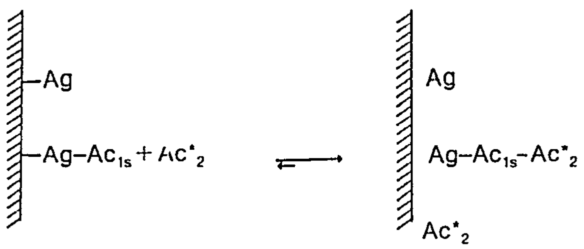


Troisième étape:

Elimination de l'excès de double anticorps marqué par lavage et comptage de la phase solide.

Ag: antigène

$Ac_{1s}$: premier anticorps spécifique de l'antigène

$Ac_{1ns}$: premier anticorps non spécifique de l'antigène

$Ac^*_2$: deuxième anticorps marqué.

Elles ont toutes en commun les caractéristiques suivantes:

- l'anticorps est marqué soit directement, soit par l'intermédiaire d'une autre molécule marquée avec laquelle il peut entrer en réaction. Ces réactifs spécifiques de l'anticorps peuvent être des «seconds anticorps» ou la protéine A de «staphilococus aureus»,
- l'un des participants à la réaction qui peut être soit l'anticorps, soit l'antigène ou l'haptène est immobilisé sur une phase solide de façon à séparer l'anticorps qui a réagi de celui qui est resté libre en solution (voir dosages I, II et III),
- l'un ou plusieurs des participants à la réaction qui peut être soit l'anticorps, soit l'antigène ou l'haptène est en large excès par rapport à un ou plusieurs autres participants. C'est pour cette raison que ces méthodes sont appelées méthodes «en excès de réactif».

En plus de la classification que nous venons d'envisager, on distingue parmi les dosages enzymoimmunologiques, deux types de dosages qui sont les dosages «homogènes» et les dosages «hétérogènes». Un dosage homogène est basé sur le fait que l'activité de la molécule marquée est modifiée lorsqu'elle est liée avec l'anticorps ou l'antigène correspondant. Dans ce cas, l'activité enzymatique du mélange réactionnel est directement reliée à la proportion de molécules marquées impliquées dans la formation du complexe antigène-anticorps. De ce fait, il n'est pas nécessaire d'effectuer une séparation et la mesure de l'activité enzymatique peut avoir lieu directement dans le milieu réactionnel.

En revanche, dans un dosage enzymoimmunologique «hétérogène», il est nécessaire d'avoir recours à un procédé de séparation, car dans ce cas, l'activité enzymatique de la molécule marquée par l'enzyme qui a participé à la formation du complexe, n'est pas modifiée. Dans ce cas, on doit séparer le complexe antigène-anticorps du milieu réactionnel pour effectuer la mesure de l'activité enzymatique du complexe, ou de l'antigène (ou anticorps) libre. C'est à cette catégorie qu'appartiennent la plupart des dosages enzymoimmunologiques développés à ce jour.

Dans tous ces dosages, l'enzyme utilisée joue un rôle très important car elle conditionne les performances du dosage, et on recherche des enzymes capables de répondre aux impératifs suivants:

1) – Disponibilité de l'enzyme à l'état pur en quantité importante, afin que l'on puisse réaliser dans de bonnes conditions la fabrication des molécules marquées d'antigène, d'haptène ou d'anticorps.

2) – Possibilité de détecter l'enzyme avec une grande sensibilité afin que l'on puisse mesurer de très faibles quantités de complexe antigène-anticorps et donc de très faibles quantités d'antigène, d'haptène ou d'anticorps dans les échantillons. Cette sensibilité de détection dépend bien entendu de la constante catalytique de l'enzyme, mais aussi de la sensibilité avec laquelle peuvent être mesurés les produits de la réaction enzymatique.

3) – Dosage enzymatique aussi simple que possible à réaliser.

4) – Présence dans l'enzyme de groupements réactifs capables de réagir avec des antigènes, des haptènes ou des anticorps pour former la molécule marquée.

5) – Stabilité de l'enzyme dans les conditions de conservation standard.

6) – Activité enzymatique de l'enzyme non identique à celle du milieu de dosage et ne présentant pas de facteurs interférant avec le dosage.

7) – Autolyse du substrat enzymatique aussi faible que possible.

A la suite de différentes recherches, on a trouvé que l'acétylcholinestérase d'Electrophorus Electricus constituait une enzyme présentant ces caractéristiques optimales pour être utilisés dans un dosage immunoenzymatique et la présente invention a précisément pour objet l'utilisation de cette enzyme pour préparer des composés qui peuvent être utilisés comme molécules marquées dans un dosage immunoenzymatique.

Le composé selon l'invention est constitué par une molécule choisie parmi les anticorps, les antigènes et les haptènes, liée par une liaison de covalence ou une liaison réversible à une enzyme et il se caractérise en ce que l'enzyme est constituée par l'acétylcholinestérase d'Electrophorus Electricus.

Grâce à l'utilisation de cette enzyme, on peut réaliser des dosages enzymoimmunologiques dans de bonnes conditions.

En effet, l'acétylcholinesterase (E.C.3.1.1.7) fait partie d'un groupe d'enzymes qui hydrolysent rapidement les esters de la choline et elle se caractérise par le fait que l'acétylcholine est son meilleur substrat. Elle est très répandue dans de nombreux tissus animaux et dans le système nerveux central et périphérique des vertébrés. On la trouve en concentration particulièrement importante dans les organes électriques de certains poissons comme Electrophorus Electricus (Gymnote) et Torpédo Marmorata (Torpille). Dans les organes électriques d'Electrophorus Electricus, l'acétylcholinestérase est 100 fois plus concentrée que dans le cerveau des oiseaux ou des mammifères. L'enzyme contenue dans ces organes électriques d'Electrophorus Electricus est rapidement et simplement purifiée en une seule étape par chromatographie d'affinité. Sa purification est beaucoup plus facile à réaliser que celle des acétylcholinestérases de Torpilles, d'oiseaux ou de mammifères qui possèdent toutes, au moins en partie, des caractéristiques hydrophobes et dont la stabilité est moindre.

L'acétylcholinestérase d'Electrophorus Electricus possède de plus une constante catalytique très élevée, et elle peut être détectée par dosage colorimétrique jusqu'à des concentrations de l'ordre de $10^{-13}$ M. Cette sensibilité de détection est nettement supérieure à celle qu'on peut observer avec les principales enzymes utilisées en enzymoimmunologie à savoir: la Peroxydase de Raifort (E.C.1.11.1.7), la β galactosidase d'Escherichia Coli (E.C.3.2.1.23) et la phosphatase alcaline d'intestin de veau (E.C.3.1.3.1).

Par ailleurs, elle possède des groupements réactifs, en particulier des groupes lysyle, tyrosyle, histidyle, tryptophanyle et glutamyle qui peuvent réagir avec certains groupements réactifs des antigènes, des haptènes ou des anticorps pour former des composés marqués utilisables en enzymoimmunologie. Enfin, cette enzyme est très stable à −20 °C ou à −173 °C pour peu qu'elle soit conservée dans un milieu adéquat.

Par conséquent, l'utilisation comme marqueur enzymatique de l'acétylcholinestérase d'Electrophorus Electricus présente de nombreux avantages aussi bien par rapport aux autres acétylcholinestérases que par rapport aux autres enzymes couramment utilisées dans le domaine de l'enzymoimmunologie.

Selon l'invention, l'acétylcholinestérase d'Electrophorus Electricus (E.C.3.1.1.7) est obtenu à partir des organes électriques d'Electrophorus Electricus et purifié par chromatographie d'affinité. On peut utiliser en particulier la méthode décrite par Bon et Coll. dans Eur. J. Biochemical 68, p. 531–539 (1976) qui permet d'obtenir environ 30 mg d'enzyme pure en quelques jours à partir d'un kilogramme d'organe électrique d'Electrophorus Electricus.

On sait que l'acétylcholinestérase existe sous plusieurs formes moléculaires nommées $A_{12}$, $A_8$, $A_4$, $G_4$, $G_2$ et $G_1$ comme cela est décrit par Bon et Coll. dans Ann. Rev. Neurosci. 1982, 5, p. 57–106. Dans les organes électriques d'Electrophorus Electricus, ce sont les formes $A_{12}$, $A_8$ et $G_4$ qui prédominent et quand l'enzyme pure est stockée, il peut se former spontanément des agrégats covalents entre les formes $A_{12}$ et $A_8$.

Selon l'invention, le composé peut être formé à partir des formes moléculaires naturelles telles que $A_{12}$, $A_8$ et $G_4$, mais aussi à partir des formes moléculaires obtenues par dégradation de ces dernières, à savoir $A_4$, $G_2$ et $G_1$ ou même à partir d'agrégats covalents entre les formes asymétriques.

Selon l'invention, la molécule constituée par un antigène ou un haptène peut être choisie parmi les médicaments tels que les analgésiques, les antibiotiques, les anticonvulsants, les agents antidiabétiques, les agent antihypertensions, les agents antinéoplastiques, les barbiturates, les dépresseurs cardiaques, les glycosides cardiaques, les hallucinogènes, les insecticides, les agents relaxants des muscles, les steroïdes, les stimulants, les alcaloïdes du tabac et les tranquillisants. Cette molécule peut aussi être constituée par une protéine du plasma ou une hormone, par exemple un polypeptide, un stéroïde ou d'autres molécules plus petites.

A titre d'exemple, les antigènes peuvent être des protéines du sérum telles que les IgG, IgE, l'haptoglobine, l'alpha-2-H-globuline, l'alpha-FP, le CEA et le PAM; les hormones peuvent être HCG, HPL, TSH, l'insuline, les oestrogènes, la progestérone, le cortisol et la thyroxine; et les médicaments peuvent être des barbiturates, des opiates, la méthadone, des amphétamines, la digoxine et le gentamycine.

La molécule peut encore être constituée par des anticorps de certains pathogènes tels que Echinococcosis granulosus, Trichinella Spiralis, Toxoplasma gondii, Treponema pallidum, les Salmonella O antigènes, Escherichia Coli, Rubella virus, Trypanosoma brucci, Trypanosoma cruzi, Schistosoma mansoni, Schistosoma haematobium, Plasmodium berghei, Plasmodium knowlesi et Vibrio cholerae. Elle peut en particulier être constituée par un anticorps monoclonal.

A titre d'exemple, nous citerons quelques résultats obtenus avec les molécules suivantes: substance P, prostaglandines, thromboxane, leukotriène, Triiodothyronine (T3), anticorps anti-IgE humaine.

Selon l'invention, pour lier par une liaison de covalence l'acétylcholinestérase d'Electrophorus Electricus à la molécule choisie parmi les antigènes, les haptènes et les anticorps, on fait réagir l'un des groupements réactifs portés par l'enzyme tel que les groupes lysyle, tyrosyle, histidyle, tryptophanyle ou glutamyle avec un groupement réactif porté par l'antigène, l'haptène ou l'anticorps,

par exemple avec une fonction acide, une fonction amine, une fonction phénol, une fonction hydroxyle ou une fonction cétone. Ceci peut être effectué en particulier par des réactions de couplage couramment utilisées, au moyen de réactifs tels que les carbodiimides, le glutaraldéhyde, la benzidine, les anhydrides mixtes, les esters actifs de l'haptène et l'acide paraaminophénylacétique.

Selon l'invention, la liaison entre l'antigène, l'haptène ou l'anticorps peut aussi être obtenue par l'intermédiaire d'une liaison non covalente résersible comme celle qui met en jeu l'avidine et la biotine ou un antigène et un anticorps.

Ainsi, le procédé de l'invention consiste à faire réagir la molécule choisie parmi les antigènes, les haptènes et les anticorps avec l'acétylcholinestérase d'Electrophorus Electricus.

Selon un mode particulier de réalisation de ce procédé, lorsque la molécule est la substance P, on prépare tout d'abord un dérivé de la substance P par réaction de celle-ci avec un ester actif de l'acide paraaminophénylacétique, et on fait réagir le dérivé ainsi obtenu avec l'acétylcholinestérase d'Electrophorus Electricus.

Selon un autre mode particulier de réalisation du procédé de l'invention, lorsque la molécule est une prostaglandine, un thromboxane ou la triiodothyronine, on prépare tout d'abord un ester actif de la prostaglandine, et on fait réagir l'ester ainsi obtenu avec l'acétylcholinestérase d'Electrophorus Electricus.

Avantageusement, l'ester actif est obtenu par réaction de l'acide libre de la molécule avec le N-hydroxysuccinimide.

Lorsque la molécule est un leukotriène, on prépare d'abord un composé intermédiaire en faisant réagir la molécule avec le dinitrofluorobenzène avant de le faire réagir avec l'acétylcholinestérase d'Electrophorus Electricus.

Lorsque la molécule est un anticorps, par exemple un anticorps anti-IgE humaine, on fixe d'abord de façon covalente de la biotine sur l'anticorps et sur l'acétylcholinestérase d'Electrophorus Electricus à l'aide d'un ester actif de la biotine.

La liaison entre l'anticorps et l'enzyme est assurée par l'avidine qui forme un complexe très stable avec les molécules de biotine.

Les composés de l'invention peuvent être utilisés en particulier comme marqueurs dans des procédés de dosage enzymoimmunologique pour déterminer la concentration en antigène, en anticorps ou en haptène d'un échantillon, en utilisant soit la technique de dosage «homogène», soit la technique de dosage «hétérogène».

Lorsqu'on utilise la technique de dosage hétérogène, la séparation entre la fraction liée et la fraction libre de la molécule marquée par l'enzyme est réalisée par des méthodes classiques, par exemple, par précipitation à l'aide d'un second anticorps. On peut utiliser aussi des phases solides constituées par un support sur lequel est immobilisé un antigène ou un anticorps. Dans ces dosages, l'activité enzymatique de l'acétylcholinestérase peut être déterminée par une méthode classique, en particulier par la méthode décrite par Ellman et Coll. dans Biochemical Pharmacology 1961, vol. 7, p. 88–95, dont la mise en œuvre est particulièrement simple et qui permet de détecter des concentrations d'enzymes de l'ordre de $10^{-3}$ M.

D'autres caractéristiques et avantages de l'invention apparaîtront mieux à la lecture des exemples suivants donnés bien entendu à titre illustratif et non limitatif en référence au dessin annexé sur lequel les figures 1 et 2 représentent les courbes d'étalonnage de dosages enzymoimmunologiques selon l'invention.

EXEMPLE 1

Préparation d'un traceur enzymatique pour le dosage immunoenzymatique de la substance P (SP).

Cette substance correspond à la séquence d'acides aminés suivante:

Arg-Pro-Lys-Pro-Gln-Gln-Phe-Phe-Gly-Leu-Met-$NH_2$.

Pour préparer le composé covalent entre la substance P et l'acétylcholinestérase d'Electrophorus Electricus, on procède en deux étapes qui consistent respectivement à préparer un dérivé intermédiaire de la substance P par conjugaison avec l'acide para-aminophénylacétique (PAPA) et à coupler le dérivé ainsi obtenu à l'enzyme par l'intermédiaire d'un sel de diazonium.

a) – Préparation du dérivé intermédiaire de la substance P.

On prépare tout d'abord un ester actif de l'acide para-aminophénylacétique (PAPA) avec la N-hydroxysuccinimide (NHS) selon le schéma réactionnel suivant:

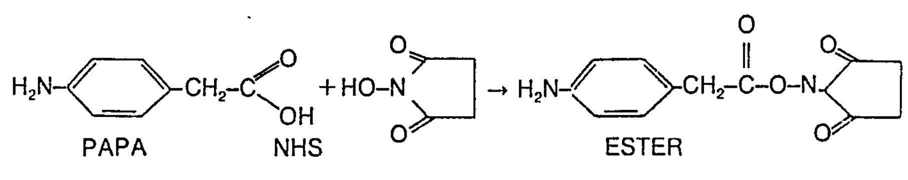


puis on fait réagir ce dérivé avec la substance P selon le schéma suivant:

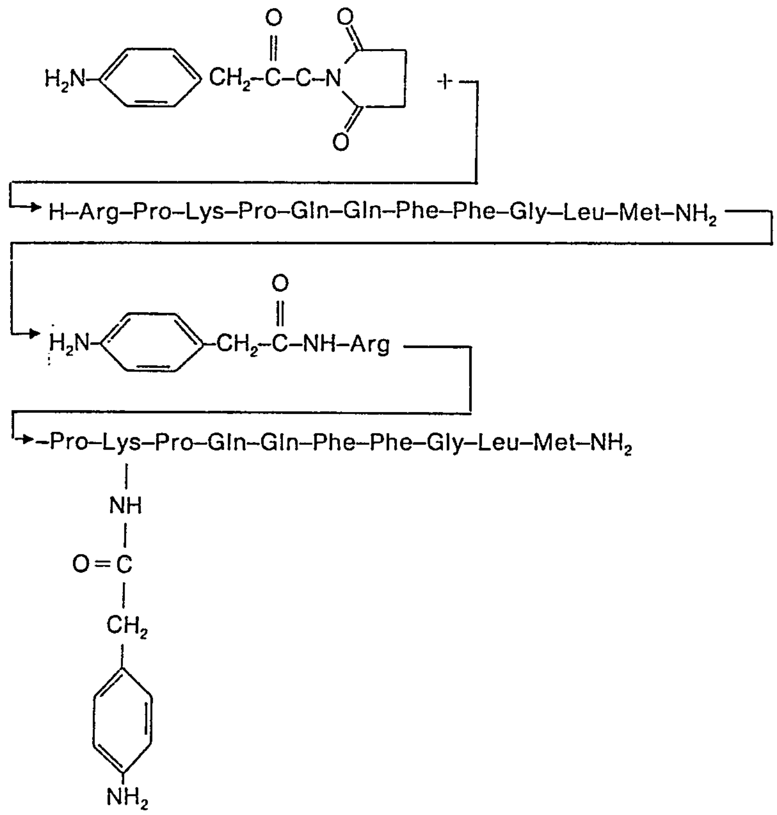

Pour préparer l'ester actif, on fait réagir 15,1 mg PAPA, 11,5 mg de NHS et 20,6 mg de dicyclocarbodiimide (DCC) dans 2 ml de diméthylformamide (DMF) pendant 18 h à 22 °C dans l'obscurité. On élimine alors le précipité formé par centrifugation, puis on ajoute 20 µl (1 µmol) du mélange réactionnel ainsi obtenu à 100 µl de DMF/tampon borate 0,1 M pH 9 (V/V) contenant 1 µmol de substance P (SP) et on laisse réagir pendant une heure à 4 °C à l'obscurité. On obtient ainsi le dérivé intermédiaire.

b) – Couplage du conjugué SP-PAPA à l'enzyme.

Cette réaction de couplage correspond au schéma suivant:

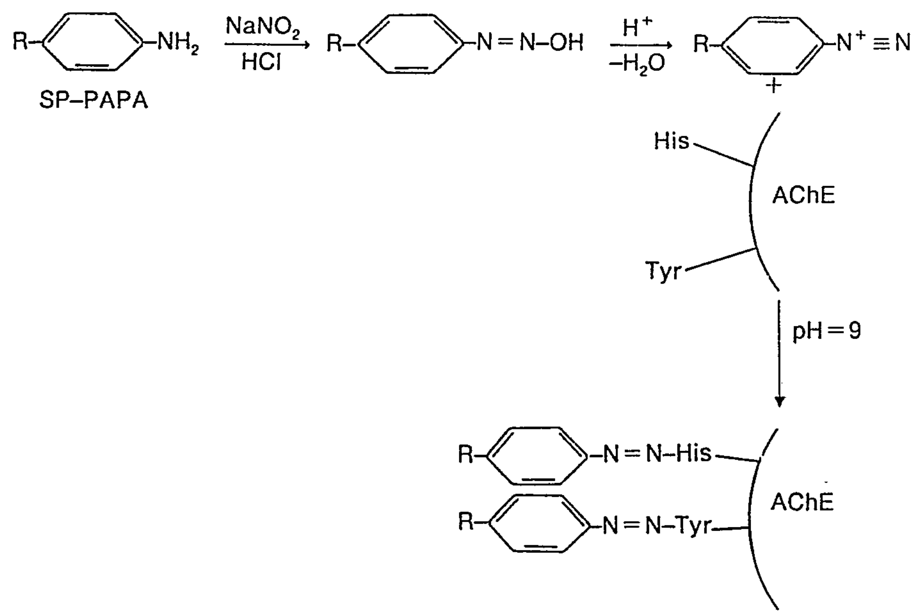


On ajoute 50 µl d'une solution aqueuse de $NaNO_2$ à 0,5% à 100 µl de HCl 1 N contenant environ 130 pmol de SP-PAPA obtenu précédemment, en opérant à 4 °C. Après une minute, on ajoute cette solution à 1 ml d'une solution d'enzyme (100 µg) dans un tampon carbonate 0,5 M (pH 9). On laisse réagir pendant 30 minutes à +4 °C à l'abri de la lumière. On neutralise l'excès de réactif SP-PAPA non couplé à l'enzyme par addition de 100 µl d'histidine (100 µg) dans le tampon carbonate. 15 minutes après, on filtre le mélange réactionnel à travers une colonne de Séphadex® (G25) de

2 cm × 40 cm en utilisant successivement comme éluant du tampon TRIS $10^{-2}$ M, du $MgCl_2$ $5.10^{-2}$ M et du NaCl 1 M (pH 7,4).

Le but de cette opération est d'éliminer la substance P non couplée à l'enzyme.

On mesure ensuite l'activité enzymatique des différentes fractions collectées et on détermine ainsi le profil d'élution de l'activité enzymatique. On réunit les fractions, puis on les distribue dans plusieurs tubes à congélation et on les conserve dans l'azote liquide. On purifie ensuite par filtration sur gel les composés correspondant aux différentes formes de l'enzyme (agrégats-formes $A_8$–$A_{12}$ et $G_4$) en utilisant le gel A15m (de Biorad) dans une colonne de 90 cm sur 1,5 cm et en éluant par le tampon TRIS précédemment utilisé. Après avoir réalisé le profil d'élution de l'activité enzymatique, on réunit les différentes fractions du pic qui correspondent à l'élution des différentes formes, et on stocke le traceur enzymatique dans l'azote liquide jusqu'à son utilisation.

EXEMPLE 2

Préparation d'un traceur enzymatique pour le dosage immunoenzymatique de la 6-keto-$PGF_{1\alpha}$ qui est un métabolite de la prostacycline.

La 6-kéto-$PGF_{1\alpha}$ correspond à la formule suivante:

6 KETO-$PGF_1\alpha$

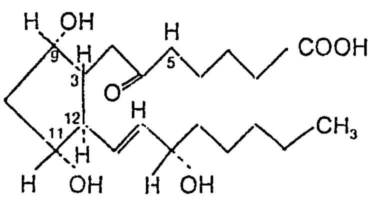

On réalise le composé en deux étapes qui consistent respectivement à former un ester actif de la 6-kéto-$PGF_{1\alpha}$ et à coupler ensuite l'ester actif avec l'enzyme.

a) – Préparation de l'ester actif.

On ajotue 10 µl de DMF contenant 5,1 µmol de N-hydroxysuccinimide (NHS) et 10 µl de DMF contenant 5,1 µmol de dicyclocarbodiimide (DCC) à 100 µl de DMF anhydre contenant 5,1 µmol de 6-kéto-$PGF_{1\alpha}$ et on laisse réagir pendant 18 heures à 22 °C à l'obscurité, ce qui conduit à la formation de l'ester actif de la 6-kéto-$PGF_{1\alpha}$ selon le schéma réactionnel suivant:

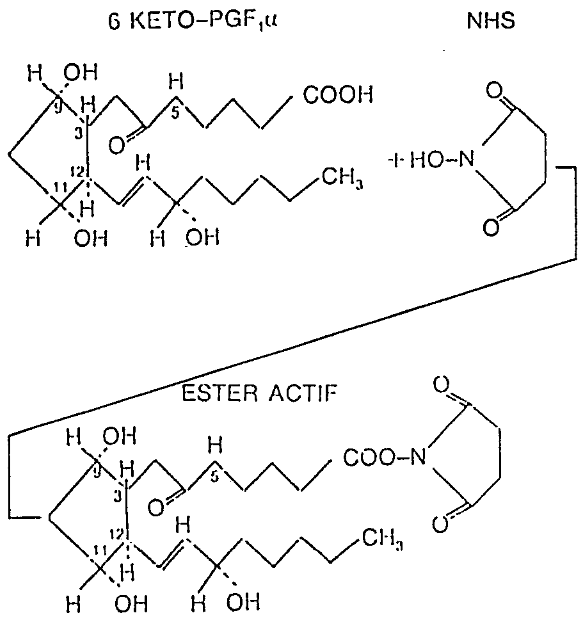


b) – Couplage de la 6-kéto-$PGF_{1\alpha}$ à l'enzyme.

On ajoute 2 µl de la solution d'ester actif dans le DMF dilué 50 fois obtenu dans l'étape a), (soit environ 10 nmol d'ester actif) à 500 µl de tampon borate 0,1 M, (pH 9) contenant 250 µg d'acétylcholinestérase. On laisse la réaction se dérouler pendant une heure à +4 °C et on neutralise l'excès d'ester par addition d'1 ml de tampon phosphate 0,1 M, NaCl 0,4 M, acide éthylène-diaminetétraacétique $10^{-3}$ M et BSA 0,5%. On obtient ainsi le couplage de l'enzyme selon le schéma réactionnel:

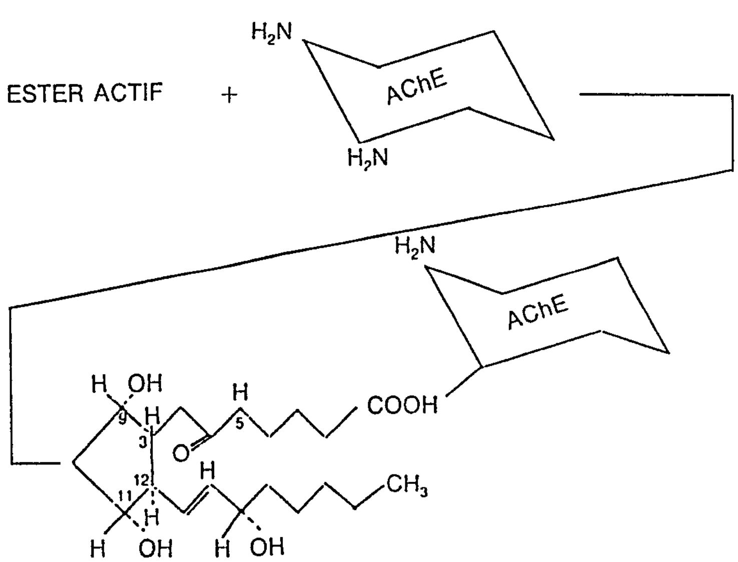

On purifie ensuite les composés correspondant aux différentes formes de l'enzyme par filtration sur gel, en suivant la méthode décrite dans l'exemple 1.

EXEMPLES 3, 4 et 5

On procède comme dans l'exemple 2 pour préparer le $PGD_2$-MO, le thromboxane B2 et la triiodothyronine marqués par l'acétylcholinestérase d'Electrophorus Electricus en utilisant les mêmes voies de synthèse.

EXEMPLE 6

Préparation d'un traceur enzymatique pour le dosage immuno-enzymatique des leukotriènes. On citera l'exemple du leukotriène $C_4$.

Le leukotriène $C_4$ ($LTC_4$) correspond à la formule suivante:

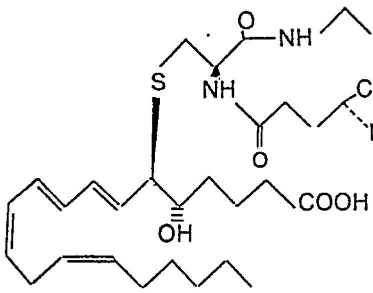

On réalise le composé en deux étapes qui consistent respectivement à former un dérivé actif du $LTC_4$ et à coupler ensuite ce dérivé avec l'enzyme.

a) – Préparation du dérivé actif du $LTC_4$.

A 100 µl de tampon phosphate contenant 50 µg de $LTC_4$ (0,1 M; pH 7,4) sont ajoutés 60 µl de méthanol contenant 200 µg de DFDB (difluorodinitrobenzène). On laisse la réaction se poursuivre pendant 30 minutes à 22 °C. On évapore le méthanol sous vide et on extrait l'excès de DFDB par 3 × 0,5 ml d'éther. L'excès d'éther est évaporé sous azote.

b) – Couplage du $LTC_4$ à l'enzyme

On ajoute 60 µl du réactif précédemment obtenu à 250 µl de tampon borate (0,1 M; pH 9) contenant environ 100 µg d'enzyme. Après 4 heures de réaction à 22 °C, on ajoute 200 µl de tampon phosphate, BSA précédemment décrit. La purification des produits de couplage est effectuée suivant la méthode décrite dans l'exemple 1 utilisant la filtration sur gel A15m (Biorad).

EXEMPLE 7

Préparation d'un traceur enzymatique pour le dosage de IgE humaine en utilisant un anticorps anti-IgE humaine marqué à l'acétylcholinestérase par l'intermédiaire du système avidine-biotine.

Le principe du couplage repose sur l'interaction très forte qui existe entre l'avidine qui est une protéine extraite du blanc d'œuf et la biotine qui est une vitamine. L'avidine a la propriété de pouvoir fixer plusieurs molécules de biotine et peut donc servir de lien entre deux molécules sur lesquelles on aura fixé au préalable de la biotine.

La biotine correspond à la formule suivante:

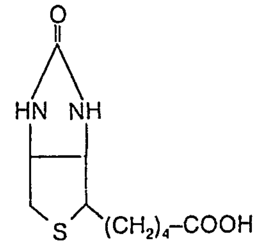

Pour marquer de façon covalente l'anticorps et l'enzyme par la biotine on utilise un ester actif de la molécule, il peut s'agir par exemple d'un ester du N-hydroxysuccinimide préparé dans des conditions similaires à celles décrites dans l'exemple 3. La réaction de l'ester avec l'anticorps et l'enzyme est aussi obtenue de la même façon que dans l'exemple 2.

Le couplage entre l'anticorps et l'enzyme est effectué au moment du dosage de la façon suivante:

– L'anticorps anti-IgE marqué à la biotine est mis à réagir avec des IgE humaines préalablement immobilisées sur un support solide par l'intermédiaire d'un autre anticorps anti-IgE non marqué.
– Après réaction et élimination de l'excès d'anticorps marqué qui n'a pas réagi, on ajoute un excès d'avidine qui se fixe sur l'anticorps marqué à la biotine.
– Après élimination de l'excès d'avidine qui n'a pas réagi, on ajoute l'acétylcholinestérase marquée par la biotine qui se fixe sur les molécules d'avidine liées à l'anticorps marqué réalisant ainsi le couplage entre l'anticorps et l'enzyme.

EXEMPLE 8

Cet exemple illustre l'utilisation du composé obtenu dans l'exemple 4 pour le dosage enzymoimmunologique du thromboxane $B_2$ ($TXB_2$).

Pour cet essai, on utilise le $TXB_2$ marqué avec les formes $A_8$–$A_{12}$ de l'acétylcholinestérase d'Electrophorus Electricus, un premier anticorps constitué par de l'antisérum de lapin anti-$TXB_2$ et un deuxième anticorps constitué par de l'antisérum de porc anti-γ-globuline de lapin. Ce dernier anticorps est immobilisé sur une phase solide.

Pour effectuer l'essai immunologique, on procède de la façon suivante:

a) – préparation du deuxième anticorps immobilisé

Dans chaque puits d'une plaque de microtitration «ImmunoNunc»® 96 F est ajouté 300 µl d'une solution d'anti-IgG de lapin purifié par chromatographie d'affinité (10 µg/ml) contenant du glutaraldéhyde à 2% dans un tampon phosphate ($10^{-2}$ M, pH 7,4). Après 12 heures d'incubation à 22 °C, on élimine la solution dans chaque puits et on ajoute 300 µl du tampon phosphate B5A (précédemment décrit).

b) – réaction enzymoimmunologique.

Le contenu de chaque puits est éliminé et on ajoute dans chaque puits 50 µl d'une solution du traceur enzymatique, 50 µl d'une solution d'anti-

corps anti $TXB_2$ et 50 µl de différentes concentrations de $TXB_2$ ou 50 µl de l'échantillon à doser.

Après 12 heures d'incubation à +4°C, le contenu de chaque puits est éliminé et on ajoute 200 µl du substrat enzymatique (réactif d'Ellman). Après 1 heure de réaction enzymatique à 22°C l'absorbance à 414 nm est mesurée dans chaque puits à l'aide du spectrophotomètre Multiskan TITERTEK® (Flow Laboratories). En se référant à la figure 1 (courbe 1) qui illustre la courbe d'étalonnage du dosage enzymoimmunologique du $TXB_2$ utilisant le composé de l'exemple 4 comme marqueur, on peut déterminer la concentration en $TXB_2$ de l'échantillon.

Sur cette figure, on a porté en abscisse la dose (pg/puits) de $TXB_2$ et en ordonnée le rapport B/Bo (en %) dans lequel B représente l'activité enzymatique liée aux anticorps en présence de $TXB_2$ et Bo représente l'activité enzymatique liée aux anticorps en l'absence de $TXB_2$. Les valeurs de liaison non spécifiques ont été soustraites et représentent moins de 5% de l'activité totale.

EXEMPLE 9

Cet exemple illustre l'utilisation du composé obtenu dans l'exemple 2 pour le dosage enzymoimmunologique du 6-keto-$PGF_{1\alpha}$. Le protocole expérimental est identique à celui décrit pour le thromboxane dans l'exemple 8. La courbe d'étalonnage se rapportant à ce dosage est représentée sur la figure 1 (courbe 2).

EXEMPLE 10

Cet exemple illustre l'utilisation du composé obtenu dans l'exemple 3 pour le dosage enzymoimmunologique de la $PGD_2$-MO. Le protocole expérimental est identique à celui décrit dans l'exemple 8. La courbe d'étalonnage se rapportant à ce dosage est représentée sur la figure 1 (courbe 3).

EXEMPLE 11

Cet exemple illustre l'utilisation du composé obtenu dans l'exemple 5 pour le dosage enzymoimmunologique de la triiodothyronine ($T_3$). Le protocole expérimental est identique à celui décrit dans l'exemple 8. La courbe d'étalonnage se rapportant à ce dosage est représentée sur la figure 2.

**Revendications**

1. Composé constitué par une molécule choisie parmi les antigènes, les haptènes et les anticorps, liée par une liaison de covalence ou une liaison réversible à une enzyme, caractérisé en ce que l'enzyme est l'acétylcholinestérase d'Electrophorus Electricus.

2. Composé selon la revendication 1, caractérisé en ce que le composé est un mélange de composés comportant l'acétylcholinestérase sous les formes moléculaires $A_8$, $A_{12}$, $G_4$ ou agrégats.

3. Composé selon l'une quelconque des revendications 1 et 2, caractérisé en ce que la molécule est une prostaglandine, un thromboxane ou un leukotriène.

4. Composé selon la revendication 3, caractérisé en ce que la prostaglandine est la 6-kéto-$PGF_{1\alpha}$ ou la $PGD_2$-MO.

5. Composé selon la revendication 3, caractérisé en ce que le thromboxane est le thromboxane $B_2$.

6. Composé selon la revendication 3, caractérisé en ce que le leukotriène est le leukotriène $C_4$.

7. Composé selon l'une quelconque des revendications 1 et 2, caractérisé en ce que la molécule est la triiodothyronine ($T_3$).

8. Composé selon l'une quelconque des revendications 1 et 2, caractérisé en ce que la molécule est un anticorps anti-IgE humaine.

9. Composé selon l'une quelconque des revendications 1 et 2, caractérisé en ce que la molécule est un anticorps monoclonal.

10. Composé selon l'une quelconque des revendications 1 et 2, caractérisé en ce que la molécule est la substance P.

11. Procédé de préparation d'un composé selon l'une quelconque des revendications 1 à 10, caractérisé en ce que l'on fait réagir la molécule choisie parmi les antigènes, les haptènes et les anticorps avec l'acétylcholinestérase d'Electrophorus Electricus.

12. Procédé de préparation d'un composé selon la revendication 10, caractérisé en ce que l'on prépare un dérivé de la substance P par réaction de celle-ci avec un ester actif de l'acide paraaminophénylacétique et en ce que l'on fait réagir le dérivé ainsi obtenu avec l'acétylcholinestérase d'Electrophorus Electricus.

13. Procédé de préparation d'un composé selon l'une quelconque des revendications 3 à 5 et 7, caractérisé en ce que l'on prépare un ester actif de la prostaglandine, du thromboxane ou de la triiodothyronine et en ce que l'on fait réagir l'ester actif ainsi obtenu avec l'acétylcholinestérase d'Electrophorus Electricus.

14. Procédé selon la revendication 13, caractérisé en ce que l'on prépare l'ester actif de la prostaglandine par réaction de celle-ci avec du N-hydroxysuccinimide.

15. Procédé de dosage enzymoimmunologique d'une molécule choisie parmi les antigènes, les haptènes et les anticorps, caractérisé en ce que l'on utilise comme traceur un composé selon l'une quelconque des revendications 1 à 10.

**Claims**

1. Compound constituted by a molecule chosen from among the antigens, haptens and antibodies, bonded by a covalent bond or a reversible bond to an enzyme, characterized in that the enzyme is the acetyl cholinesterase of Electrophorus Electricus (electric eel).

2. Compound according to claim 1, characterized in that the compound is a mixture of compounds containing acetyl cholinesterase in the molecular forms $A_8$, $A_{12}$, $G_4$ or aggregates.

3. Compound according to either of the claims 1 and 2, characterized in that the molecule is a prostaglandin, a thromboxan or a leucotrien.

4. Compound according to claim 3, characterized in that the prostaglandin is 6-keto-$PGF_{1\alpha}$ or $PGD_2$-MO.

5. Compound according to claim 3, characterized in that the thromboxan is thromboxan $B_2$.

6. Compound according to claim 3, characterized in that the leucotrien is leucotrien $C_4$.

7. Compound according to either of the claims 1 and 2, characterized in that the molecule is triiodothyronin ($T_3$).

8. Compound according to either of the claims 1 and 2, characterized in that the molecule is a human anti IgE antibody.

9. Compound according to either of the claims 1 and 2, characterized in that the molecule is a monoclonal antibody.

10. Compound according to either of the claims 1 and 2, characterized in that the molecule is substance P.

11. Process for the preparation of a compound according to any one of the claims 1 to 10, characterized in that the molecule chosen from among the antigens, haptens and antibodies is reacted with electric eel acetyl cholinesterase.

12. Process for the preparation of a compound according to claim 10, characterized in that a derivative of substance P is prepared by reacting the latter with an active ester of paraaminophenylacetic acid and wherein the thus obtained derivative is reacted with electric eel acetyl cholinesterase.

13. Process for the preparation of a compound according to any one of the claims 3 to 5 and 7, characterized in that an active ester of prostaglandin, thromboxan or triiodothyronin is prepared and the thus obtained active ester is reacted with electric eel acetyl cholinesterase.

14. Process according to claim 13, characterized in that the active ester of prostaglandin is prepared by reacting the latter with N-hydroxysuccinimide.

15. Process for the enzymoimmunological determination of a molecule chosen from among the antigens, haptens and antibodies, characterized in that the tracer used is a compound according to any one of the claims 1 to 10.

**Patentansprüche**

1. Verbindung, bestehend aus einem Molekül, das ausgewählt wird aus Antigenen, Haptenen und Antikörpern, das mit einer kovalenten Bindung oder mit einer reversiblen Bindung mit einem Enzym verbunden ist, dadurch gekennzeichnet, dass es sich bei dem Enzym um die Acetylcholinesterase von «Elektrophorus Electricus» handelt.

2. Verbindung nach Anspruch 1, dadurch gekennzeichnet, dass es sich bei der Verbindung um ein Gemisch von Verbindungen handelt, das umfasst Acetylcholinesterase in den Molekülformen $A_8$, $A_{12}$, $G_4$ oder Aggregate.

3. Verbindung nach einem der Ansprüche 1 und 2, dadurch gekennzeichnet, dass es sich bei dem Molekül um ein Prostaglandin, ein Thromboxan oder ein Leukotrien handelt.

4. Verbindung nach Anspruch 3, dadurch gekennzeichnet, dass es sich bei dem Prostaglandin um 6-Keto-$PGF_{1\alpha}$ oder $PGD_2$-MO handelt.

5. Verbindung nach Anspruch 3, dadurch gekennzeichnet, dass es sich bei dem Thromboxan um das Thromboxan $B_2$ handelt.

6. Verbindung nach Anspruch 3, dadurch gekennzeichnet, dass es sich bei dem Leukotrien um das Leukotrien $C_4$ handelt.

7. Verbindung nach einem der Ansprüche 1 und 2, dadurch gekennzeichnet, dass es sich bei dem Molekül um Trjodthyronin ($T_3$) handelt.

8. Verbindung nach einem der Ansprüche 1 und 2, dadurch gekennzeichnet, dass es sich bei dem Molekül um einen Anti-IgE-Human-Antikörper handelt.

9. Verbindung nach einem der Ansprüche 1 und 2, dadurch gekennzeichnet, dass es sich bei dem Molekül um einen monoklonalen Antikörper handelt.

10. Verbindung nach einem der Ansprüche 1 und 2, dadurch gekennzeichnet, dass es sich bei dem Molekül um die Substanz P handelt.

11. Verfahren zur Herstellung einer Verbindung nach einem der Ansprüche 1 bis 10, dadurch gekennzeichnet, dass man das Molekül, ausgewählt aus Antigenen, Haptenen und Antikörpern, mit der Acetylcholinesterase von «Electrophorus Electricus» reagieren lässt.

12. Verfahren zur Herstellung einer Verbindung nach Anspruch 10, dadurch gekennzeichnet, dass man ein Derivat der Substanz P herstellt zur Umsetzung derselben mit einem aktiven Ester der p-Aminophenylessigsäure, und dass man das so erhaltene Derivat mit der Acetylcholinesterase von «Electrophorus Electricus» reagieren lässt.

13. Verfahren zur Herstellung einer Verbindung nach einem der Ansprüche 3 bis 5 und 7, dadurch gekennzeichnet, dass man einen aktiven Ester von Prostaglandin, Thromboxan oder Trijodthyronin herstellt und dass man den so erhaltenen aktiven Ester mit der Acetylcholinesterase von «Electrophorus Electricus» reagieren lässt.

14. Verfahren nach Anspruch 12, dadurch gekennzeichnet, dass man den aktiven Ester von Prostaglandin herstellt durch Umsetzung desselben mit N-Hydroxysuccinimid.

15. Verfahren zur enzymimmunologischen Dosierung eines Moleküls, ausgewählt aus Antigenen, Haptenen und Antikörpern, dadurch gekennzeichnet, dass man als Tracer eine Verbindung nach einem der Ansprüche 1 bis 10 verwendet.

FIG.1

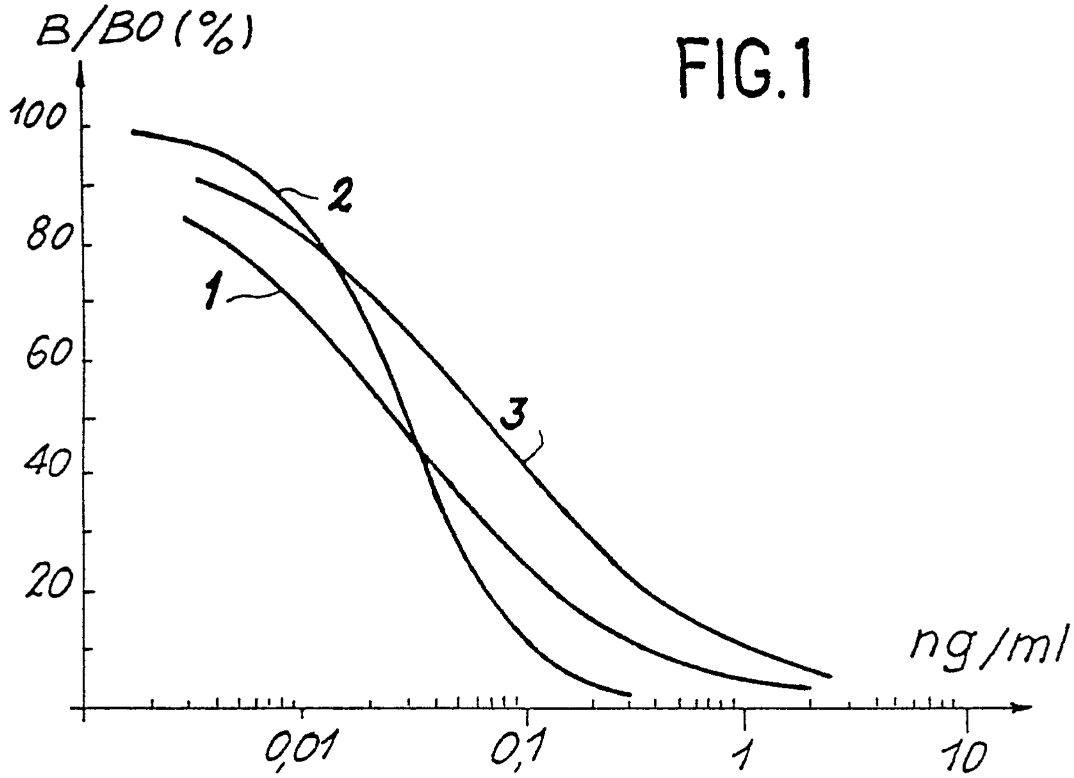
B/B0(%)
100
80
60
40
20
1
2
3
ng/ml
0,01
0,1
1
10

FIG.2

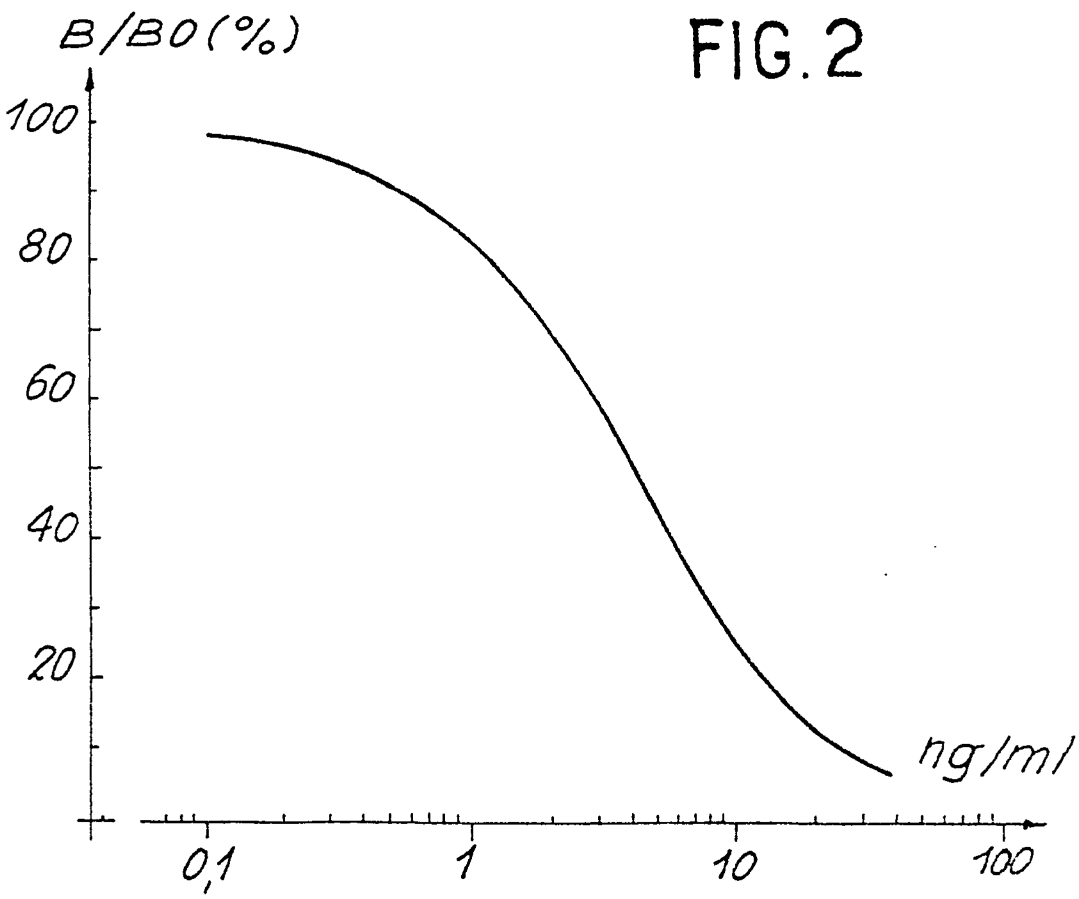
B/B0(%)
100
80
60
40
20
ng/ml
0,1
1
10
100